# EUROPÄISCHE PATENTSCHRIFT

(11) **EP 1 560 537 B1**
(45) Veröffentlichungstag und Bekanntmachung des Hinweises auf die Patenterteilung: **14.05.2008**
(21) Anmeldenummer: 03767385.2
(22) Anmeldetag: 27.10.2003
(51) Int. Cl.: A61C 1/08, A61B 17/17

(54) **VERFAHREN ZUR LAGERICHTIGEN HERSTELLUNG EINER KAVITÄT, INSBESONDERE EINER KNOCHENKAVITÄT UND INSTRUMENT HIERFÜR**
METHOD FOR PRECISELY-POSITIONED PRODUCTION OF A CAVITY, ESPECIALLY A BONE CAVITY AND INSTRUMENT THEREFOR
PROCEDE POUR PRODUIRE UNE CAVITE, EN PARTICULIER UNE CAVITE OSSEUSE, A UN EMPLACEMENT PRECIS ET INSTRUMENT ADAPTE A CET EFFET

(30) Priorität: 25.10.2002 DE 10250006
(43) Veröffentlichungstag der Anmeldung: 10.08.2005
(73) Patentinhaber: Sirona Dental Systems GmbH, 64625 Bensheim (DE)
(72) Erfinder: SALIGER, Günter, 64625 Bensheim (DE)
(74) Vertreter: Sommer, Peter
(86) Internationale Anmeldenummer: PCT/DE2003/003571
(87) Internationale Veröffentlichungsnummer: WO 2004/039278

(56) Entgegenhaltungen:
- EP-A- 0 868 886
- DE-A- 4 009 438
- US-A- 5 049 070
- US-A- 5 429 502
- US-B1- 6 419 484

## Beschreibung

### Technisches Gebiet

Die Erfindung betrifft ein Verfahren zur lagerichtigen Positionierung eines Handinstruments an einer. Präparationsstelle und eine Kombination hierfür.

In der zahnärztlichen Implantologie geht es darum, körperfremde Passstücke, sogenannte Implantate in den Knochen einzugliedern, um auf diesem Weg eine substanzschonende, prothetische Versorgung von Zahnlücken und eine Versorgung von zahnlosen Kiefern mit festsitzendem Zahnersatz zu ermoglichen.

Bei der Implantation dieser Passstücke müssen verschiedene zahnärztliche und anatomische Randbedingungen eingehalten werden, um einen langfristigen Behandlungserfolg zu erzielen. Die Übertragung der Kaukräfte in den Kieferknochen muss so geschehen, dass die anerkannten Regeln der Biomechanik und der zahnärztlichen Prothetik Beachtung finden. Körperhöhlen dürfen nicht eröffnet und Nervenbahnen dürfen nicht verletzt werden. Schließlich muss auf ein quantitativ und qualitativ ausreichendes Angebot an Knochensubstanz geachtet werden.

Diese Randbedingungen erfordern es, eine umfassende Diagnostik vor dem Eingriff und eine angemessene Planung des Eingriffs selbst durchzuführen. Schlussendlich gilt es eine gute Umsetzung der Planung während der Operation zu erreichen.

### Stand der Technik

Im Regelfall kann der Implantologe mit den heute schon verfügbaren technischen Hilfsmitteln wie Computertomographie (CT), Orthopantomographie (OPG), Messung der Knochendichte und -dicke eine ausreichende Diagnostik und damit eine gute Planung von Implantatversorgungen durchführen.

Im Bereich der Umsetzung dieser Planung in die Realität ergeben sich jedoch erhebliche Defizite.

In den letzten Jahren wurde der Versuch unternommen, diese Lücke zu schließen. Dabei kamen Verfahren der Neurochirurgie und der Orthopädie zum Einsatz. An dem Patienten und an den Operationsinstrumenten werden Markierungen angebracht, die mit Hilfe zweier Kameras beobachtet werden. Aus der bekannten Geometrie der Operationsinstrumente und der Markierungen lässt sich die relative Position z. B. eines Bohrinstrumentes ermitteln. Nach diesem Prinzip kann man dann eine effektive Umsetzung der Implantatplanung bewerkstelligen. Dabei erlaubten es solche Verfahren, Genauigkeiten von 1 mm zu erzielen, d. h. es können Abweichungen zwischen der geplanten und der realisierten Position in der Größe von 1 mm auftreten.

Der Nachteil dieser Geräte ist allerdings ihr vergleichsweise hoher Preis. Damit sind diese Geräte nur dann wirtschaftlich sinnvoll einsetzbar, wenn eine Vielzahl von Implantaten gesetzt wird.

Das Ziel der Erfindung ist es nun, eine kostengünstigere Lösung für das oben beschriebene Problem anzugeben, um damit bereits den wirtschaftlichen Einsatz bei nur wenigen Implantaten zu erreichen.

### Darstellung der Erfindung

Der hier beschriebenen Erfindung gemäß Anspruch 1 und 2 liegt die Idee zugrunde, dass geeignete Oberflächenmerkmale, wie beispielsweise die Horizontlinie einer topographischen Oberfläche in kodierter Form auch die Position des Beobachters enthält.

Ausgehend von dieser Tatsache wird ein Verfahren zur lagerichtigen Positionierung eines für eine Bearbeitung ausgebildeten Handinstruments an einer Präparationsstelle vorgeschlagen, bei dem unter Verwendung eines Handinstruments eine Berechnung positionsabhängiger Oberflächenmerkmalen aus einem dreidimensionalen Datensatz der Oberfläche der Präparationsstelle zu einer gewünschten Position eines in die Kavität einzubringenden Implantats erfolgt, wobei der Bereich, in dem die Kavität erstellt und anschließend das Implantat eingebracht werden soll, als dreidimensionaler Datensatzes von Volumendaten vorliegt.

Mittels einer am Handinstrument angeordneten Kamera mit vorgegebenem Abstand zu einem Bearbeitungswerkzeug wird mindestens ein ein tatsächlich sichtbares positionsabhängigen Oberflächenmerkmal aufweisender Teilausschnitt der Präparationsstelle erfasst und als Videobild angezeigt. In das Videobild wird das berechnete positionsabhängige Oberflächenmerkmal für die Sollposition des Handinstruments eingeblendet, wobei sich durch Veränderung der Position und der Neigung des Handinstruments das eingeblendete positionsabhängige Oberflächenmerkmal bezüglich seiner Lage zu dem tatsächlich sichtbaren positionsabhängigen Oberflächenmerkmal verändert und insbesondere zur Deckung gebracht werden kann.

Unter der Bezeichnung "Kamera" werden alle Messeinheiten, Abbildungseinheiten oder Bilderfassungseinheiten verstanden, die dazu in der Lage sind, einen Teilausschnitt der Präparationsstelle so zu erfassen, dass eine Anzeige als Videobild möglich ist.

Dadurch ist es möglich, dem Benutzer vor der eigentlichen Bearbeitung anzuzeigen, ob die Orientierung seines Handinstruments derart ist, dass der gewünschte Verlauf der Kavität erzeugt und das Implantat an der geplanten Stelle inseriert werden kann.

Über spezielle Markierungen, sogenannte röntgenopake Marker, die sowohl in den Volumendaten des Röntgenbilds als auch im 3D-Datensatz der Oberfläche sichtbar sind, ist die Relativlage von Röntgenaufnahme und 3D-Datensatz bekannt. Die Einbaulage des Implantats wird anhand der Röntgenaufnahme durch den Implantologen festgelegt. Damit ist ihre Lage auch relativ zum 3D-Datensatz bekannt und von dieser Position wird mittels des 3D-Datensatzes das positionsabhängige Oberflächenmerkmal berechnet, das sich ergäbe, wenn man das Bohrinstrument an die durch die Planung definierte Position bringt und es entsprechend der geplanten Bohrachse ausrichtet.

Als Oberflächenmerkmal wird eine Horizontlinie verwendet.

Ein weiterer Gegenstand der Erfindung gemäß Anspruch 2 ist eine Kombination aus einem Handinstrument, einer Anzeige und einer Auswerteeinheit zur Herstellung oder Bearbeitung von Kavitäten, insbesondere von Knochenkavitäten, welches ein Bearbeitungswerkzeug umfasst. Am Handinstrument ist eine Kamera vorgesehen, deren Abstand zur Spitze des Bearbeitungswerkzeuges vorgegeben ist. Die Anzeige zeigt von der Auswerteeinheit bereitgestellte Daten in Form einer von der Auswerteeinheit berechneten Horizontlinie an und ist mit dem Handinstrument verbunden.

Mit einer die weiteren Merkmale des Anspruchs 2 aufweisenden Kombination ist die Erfassung der Präparationsstelle und der Vergleich mit einem dreidimensionalen Datensatz von Volumendaten oder von Oberflächendaten möglich.

Vorteilhafterweise besitzt die Kamera eine Schärfentiefe von 5 bis 30 mm und erfasst eine Rundumsicht. Dadurch ist eine Orientierung nach der Seite mit den am stärksten ausgeprägten Orientierungsmerkmalen des 3D-Datensatzes möglich.

Gemäß einer Weiterbildung ist die Videokamera in das dem Bearbeitungswerkzeug zugewandte Ende des Instruments integriert. Dadurch ist die genaue Erfassung der tatsächlichen Horizontlinie des Vergleichsbildes an der Präparationsstelle möglich.

Schließlich können Leuchtmittel vorgesehen sein, welche den für die Erfassung und Darstellung der Horizontlinie relevanten Teil oder andere markante Oberflächenmerkmale der Oberfläche ausleuchten.

Weitere vorteilhafte Ausgestaltungen werden im Ausführungsbeispiel beschrieben.

### Kurzbeschreibung der Zeichnung

Das erfindungsgemäße Verfahren wird anhand der Zeichnung erläutert. Es zeigen:
- Fig. 1: Darstellung einer Horizontlinie in einer ersten Ansicht,
- Fig. 2: Darstellung einer Horizontlinie in einer zweiten Ansicht,
- Fig. 3: Anzeige eines Zahns und einer Soll-Horizontlinie mit Überdeckung,
- Fig. 4,7: korrekte Platzierung des Bearbeitungswerkzeugs des Handinstruments,
- Fig. 5: Anzeige eines Zahns und einer Soll-Horizontlinie ohne Überdeckung,
- Fig. 6: abweichende Platzierung des Bearbeitungswerkzeugs des Handinstruments,
- Fig. 8: die Horizontlinie vor der Bearbeitung in einer Standposition,
- Fig. 9: eine Vorrichtung zur Durchführung des Verfahrens als Schema.

### Ausführungsbeispiel der Erfindung

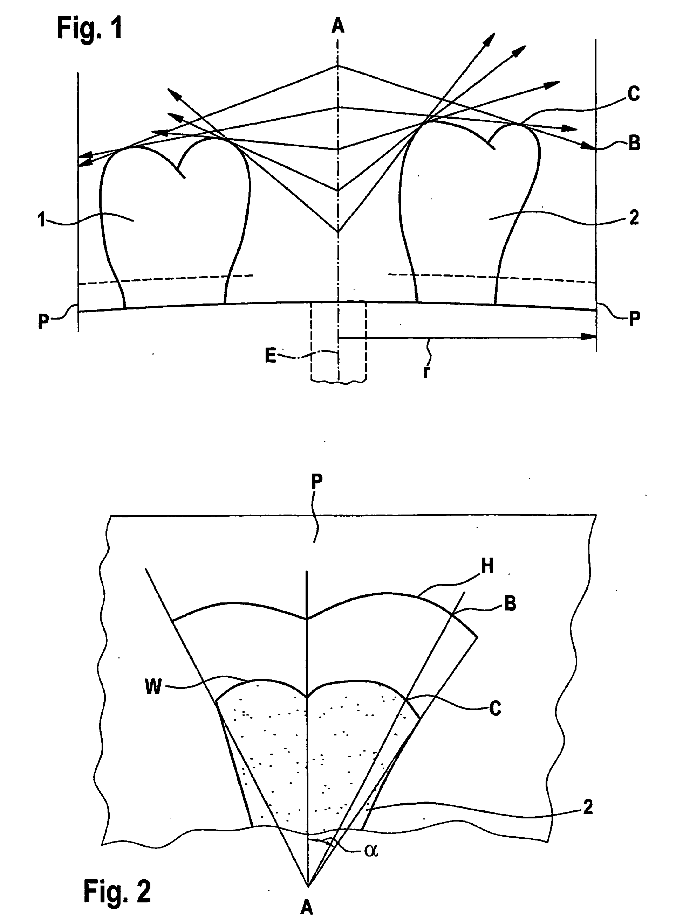

Der Begriff der Horizontlinie als Beispiel eines positionsabhängigen Oberflächenmerkmals ist anhand der Fig. 1 und 2 erläutert. Eine Horizontlinie ist durch die nachfolgende Abbildungsvorschrift gekennzeichnet. Ein Beobachter an einem Ort A baut um sich herum einen z.B. kreisförmigen Projektionsschirm P auf, der mit einer transparenten Folie bespannt ist und dessen Radius r bekannt ist. Nun wird für jedes Winkelelement alpha des gesamten Panoramas die Stelle B auf dem Schirm P markiert, an der Horizont C für den im Zentrum A befindlichen Beobachter zu liegen scheint. Die sich so ergebende Abbildung wird als Horizontlinie H bezeichnet. Kennzeichen der Horizontlinie H ist, dass in ihrer Form implizit auch der Standort des Beobachters kodiert wird. Umkehrt kann man in Kenntnis einer bestimmten hinreichend eigenartigen Horizontlinie den Ort wiederfinden, von dem aus sie beobachtet wurde.

Ob es zwischen der Horizontlinie und den abgebildeten Objekten eine eindeutige Beziehung gibt, hängt von den abgebildeten Objekten ab. Je komplexer die Objekte sind, um so eindeutiger ist die Beziehung. Folgendes Beispiel verdeutlicht diesen Sachverhalt: Aus einem Bild, das eine Bergkette zeigt, kann zusammen mit der Information, welcher Weg im Gebirge abgeschritten wurde, auf den Ort geschlossen werden, an dem dieses Bild aufgenommen wurde.

Die Ortsbeziehung zwischen den Messdaten einer Oberflächenvermessung und den per Röntgen hergestellten Volumendaten ist beispielsweise über röntgenopake Marker bekannt. Die Einbaulage des Implantats wird anhand einer Röntgenaufnahme (OPG, CT) ermittelt. Die dazu bekannte Lage der Oberflächendaten wird während der Herstellung der Kavität zur Kontrolle der lagerichtigen Ausrichtung des Werkzeugs benutzt.

Dabei werden die Horizontlinien der Nachbarzähne 1, 2 oder sonstige geeignete und positionsabhängige Oberflächenmerkmale berücksichtigt.

Längs der Einschubrichtung des Implantat werden die zu erwartenden Horizontlinien mit Hilfe des 3D-Datensatzes berechnet. Dabei liegen alle Orte A auf einer Achse E parallel, insbesondere aber deckungsgleich mit der Einschubachse.

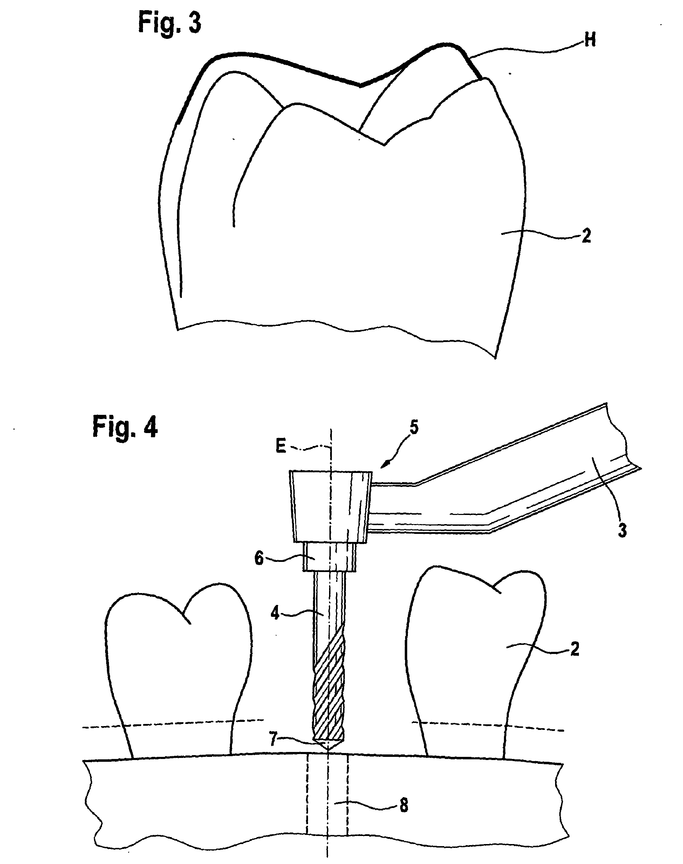

Fig. 3 zeigt schematisch einen Zahn 1, wie er durch die Optik des Bearbeitungsinstruments erfasst wird und auf einer Anzeige erscheint. Bei der fett markierten Linie handelt es sich um die Horizontlinie H, die an der durch die Planung definierten Solllage der Implantatachse sichtbar wäre. Wirklicher Horizont und Horizontlinie sind hier deckungsgleich. Die augenblicklich eingenommene Position des Bohrers ist daher korrekt.

Fig. 4 zeigt ein Bearbeitungsinstrument 3 mit einem Werkzeug 4, der sich an der vorausbestimmten Stelle befindet. Das Bearbeitungsinstrument 3 weist an seinem dem Bearbeitungswerkzeug 4 zugewandten Ende 5 eine in einem kleinen zylindrischen Aufsatz 6 untergebracht Optik zur Erfassung der Umgebung auf, etwa eine integrierte intraorale Videokamera. Die Relativlage zwischen der Spitze 7 des Werkzeugs und der Position der Optik ist bekannt. Der Aufsatz 6 befindet sich oberhalb des Werkzeugs 4 und enthält die Optik und ein Faserbündel zur Beleuchtung der Zähne.

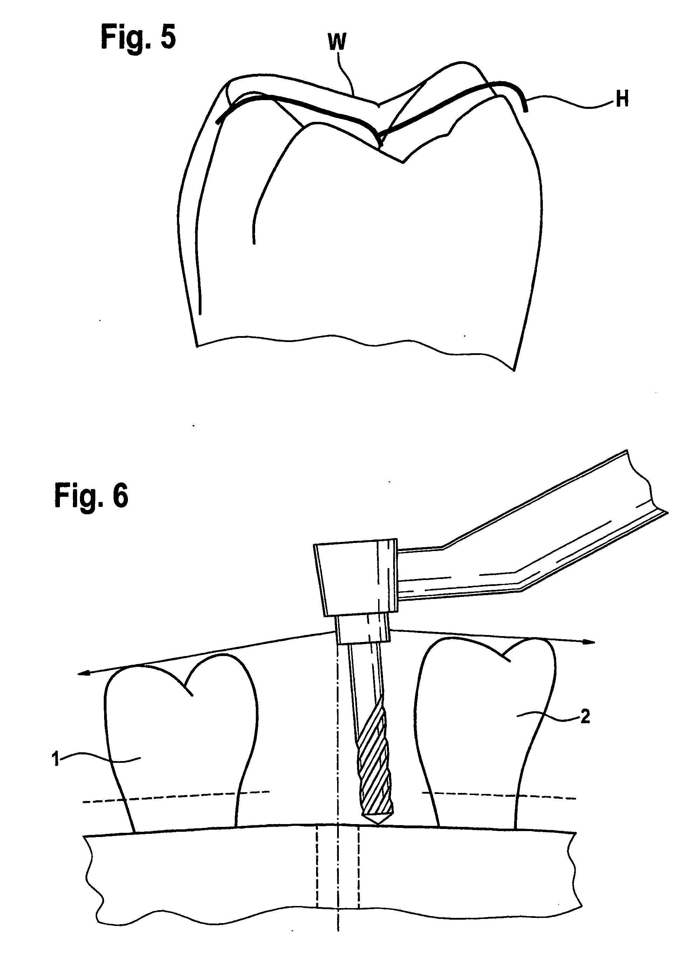

Die Optik sollte einen Schärfentiefebereich zwischen 5 mm und 30 mm aufweisen. Sie darf nicht telezentrisch sein, d. h. Objekte in verschiedenen Entfernungen müssen unterschiedlich groß abgebildet werden. Es kann sich um eine 360 Grad Panoramaoptik handeln, wie es in der Fig. 6 angedeutet ist. Es ist jedoch ausreichend, wenn ein Bildfeld verwendet wird in einer Größe, dass ein Zahn erfasst wird. Die Kamera erfasst dann den Zahn 1, 2 also nur aus einer Richtung.

Dabei werden die Nachbarzähne 1 und 2 im Aufpunkt des herzustellenden Implantatbohrlochs 8 vollständig abgebildet. Wichtig bei dem Verfahren ist, dass sich ähnlich wie bei Bergkuppen die Höcker der Zähne hervorheben. Andere markante Oberflächenmerkmale können beispielsweise durch Füllungen bereitgestellt werden. Auch hier sind grundsätzlich alle sichtbaren, positionsabhängigen Oberflächenmerkmale umfasst.

An die Verzeichnung der Optik sind keine besonderen Anforderungen zu stellen. Ein Schwarzweiß-Bild ist grundsätzlich ausreichend.

Fig. 5 zeigt schematisch eine Anzeige, bei der der wirkliche Horizont W und die Horizontlinie H nicht deckungsgleich sind. Die augenblicklich eingenommene Position des Bohrers ist nicht korrekt, siehe Fig. 6. Der Bohrer befindet sich noch nicht an der vorausbestimmten Stelle.

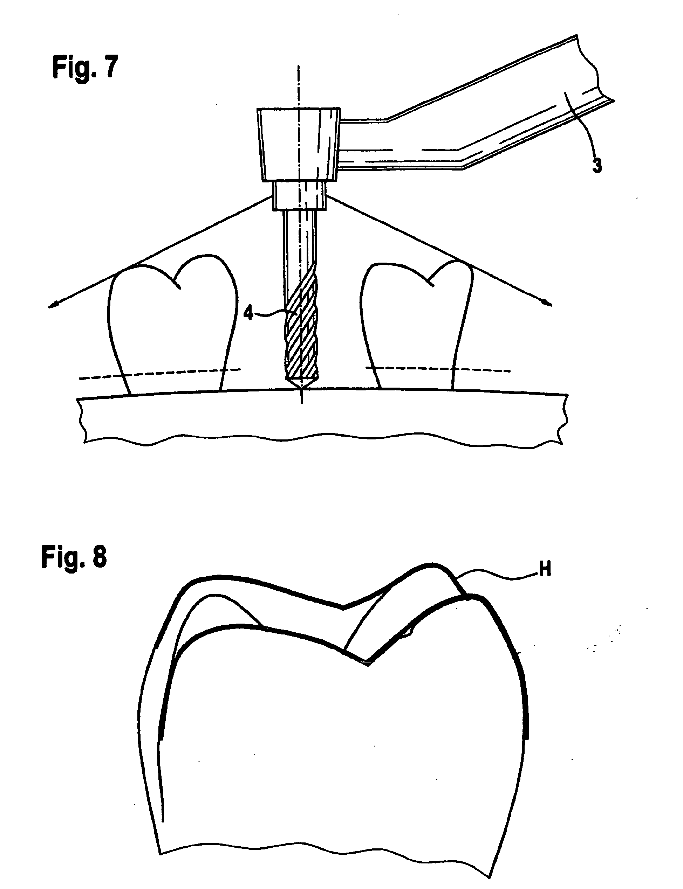

Fig. 7 zeigt das lagerichtig aufgesetzte Werkzeug 4 des Bearbeitungsinstruments 3. Fig. 8 zeigt die zugehörige Horizontlinie. Die hintere passende Horizontlinie H zeigt, dass der Aufpunkt der Bohrung lagerichtig gefunden wurde.

Insgesamt läuft das Verfahren wie folgt ab: Die Zahn- bzw. Kieferoberfläche wird mit röntgenopaken Markierungen versehen, die eine Korrelation von 3D-Messdaten eines Volumens aus Röntgenbildern mit 3D-Meßdaten der Oberfläche erlauben. Die röntgenopaken Marker sind sowohl im Röntgenbild wie auch im Oberflächenbild sichtbar. Sie erlauben es, die Relativlage der beiden Aufnahmen zueinander zu bestimmen.

Anhand der Röntgenbilder wird eine Planung der Implantate durchgeführt. Dabei wird die Einsetztiefe und die Winkellage des Bohrkanals bestimmt.

Für die Startpositionen des Bearbeitungswerkzeugs am Anfang des herzustellenden Bohrkanals wird aus den 3D-Messdaten der Oberfläche die zugehörige Horizontlinie berechnet.

Das Instrument zur Herstellung der Implantatbohrung besitzt eine intraorale Videokamera, deren Entfernung zu der Spitze des Bohrinstrumentes bekannt ist. In das Videobild dieser Kamera wird unter Berücksichtigung aller optischen Verzerrungen die berechnete Horizontlinie für die Sollposition eingeblendet. Durch Veränderung der Position und der Neigung des Bohrinstrumentes kann die eingeblendete Horizontlinie mit dem tatsächlich sichtbaren Horizont zur Deckung gebracht werden.

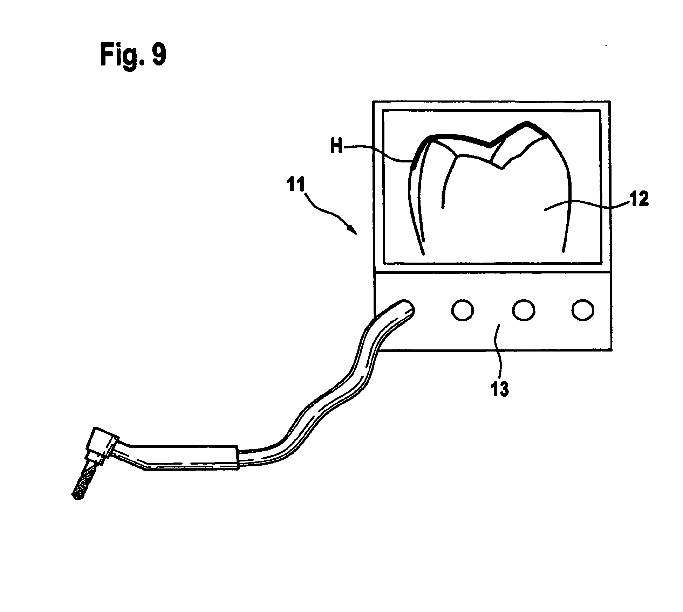

In Fig. 9 ist ein Aufbau schematisch dargestellt. Die Einheit 11 umfasst eine Anzeige 12, auf dem Handinstrument 3 aufgenommenen Bilder dargestellt werden können. Gleichzeitig dient eine Auswerteeinheit 13 der Steuerung des Bohrantriebs und der Berechnung des Oberflächenmerkmals, hier der Horizontlinie. Die Steuerung des Instrumente kann so erfolgen, dass dann, wenn eine Abweichung von der vorgegebenen Position festgestellt wird, der Antrieb für das Bearbeitungswerkzeug abgeschaltet wird und erst dann wieder freigegeben wird, wenn die richtige Position wieder erreicht wird.

### Bezugszeichenliste

- 1: Zahn
- 2: Zahn
- 3: Handinstrument
- 4: Werkzeug
- 5: Ende des Handinstruments
- 6: Aufsatz
- 7: Spitze des Werkzeugs 4
- 8: Bohrloch
- 9:
- 10:
- 11: Einheit
- 12: Anzeige
- 13: Auswerteeinheit

- A: Ort/Zentrum des Beobachters
- B: Stelle
- C: Horizont
- E: Achse
- H: Horizontlinie
- H': Horizontlinie
- P: Projektionsschirm
- r: Radius
- W:

## Patentansprüche

1. Verfahren zur lagerichtigen Positionierung eines ein Bearbeitungswerkzeug aufweisenden Handinstruments an einer Präparationsstelle, umfassend folgende Schritte, die alle vor der eigentlichen Bearbeitung liegen:
- Berechnung von positionsabhängigen Oberflächenmerkmalen aus einem dreidimensionalen Datensatz der Oberfläche der Präparationsstelle zu einer gewünschten Position eines in die Kavität einzubringenden Implantats, wobei der Bereich, in dem die Kavität erstellt werden soll, als dreidimensionaler Datensatz von Volumendaten vorliegt;
- Erfassen mindestens eines ein tatsächlich sichtbares Oberflächenmerkmal aufweisenden Teilausschnittes der Präparationsstelle mittels einer am Handinstrument angeordneten Kamera mit vorgegebenem Abstand zu einem Bearbeitungswerkzeug und Anzeige als Videobild;
- Einblenden eines berechneten Oberflächenmerkmals für die Sollposition des Handinstruments, wobei durch Veränderung der Position und der Neigung des Handinstruments das eingeblendete Oberflächenmerkmal bezüglich seiner Lage zu dem tatsächlich sichtbaren Oberflächenmerkmal verändert, insbesondere zur Deckung gebracht werden kann, wobei als Oberflächenmerkmal eine Horizontlinie verwendet wird.

2. Kombination aus einem Handinstrument (3), einer Anzeige (12) und einer Auswerteeinheit (13) zur Herstellung oder Bearbeitung von Kavitäten, insbesondere von Knochenkavitäten, wobei das Handinstrument (3) ein Bearbeitungswerkzeug (4) und eine Kamera (6) umfasst, deren Abstand zur Spitze (7) des Bearbeitungswerkzeugs (4) vorgegeben ist, und wobei das Handinstrument (3) mit der Anzeige (12) verbunden ist, und wobei mindestens ein ein tatsächlich sichtbares Oberflächenmerkmal aufweisender Teilausschnitt der Präparationsstelle mittels der Kamera (6) erfasst und auf der Anzeige (12) als Videobild dargestellt ist, **dadurch gekennzeichnet, dass** die Anzeige (12) von der Auswerteeinheit (13) bereitgestellte Daten in Form einer von der Auswerteeinheit (13) berechneten Horizontlinie (H) anzeigt und dass durch Veränderung der Position und der Neigung des Handinstruments (3) die eingeblendete Horizontlinie (H) bezüglich ihrer Lage zu der tatsächlich sichtbaren Horizontlinie veränderbar ist, insbesondere zur Deckung gebracht werden kann.

3. Kombination nach Anspruch 2, **dadurch gekennzeichnet, dass** die Kamera (6) eine Schärfentiefe von 5 bis 30 mm besitzt und eine Rundumsicht erfasst.

4. Kombination nach Anspruch 2 oder 3, **dadurch gekennzeichnet, dass** die Kamera (6) in das dem Bearbeitungswerkzeug (4) zugewandten Ende (5) des Instruments (3) integriert ist.

5. Kombination nach einem der Ansprüche 2 bis 4, **dadurch gekennzeichnet, dass** Leuchtmittel vorgesehen sind, welche den für die Erfassung und Darstellung der Horizontlinie relevanten Teil der Oberfläche ausleuchten.

## Claims

1. A method for correctly positioning a hand instrument comprising a machining tool, at a preparation site, comprising the following steps, all of which are to be carried out prior to commencement of machining:
- computing position-dependent surface features of a three-dimensional data set relating to the surface of the preparation site relative to a desired position of an implant to be inserted into the cavity to be drilled, the area in which the cavity is to be created existing in the form of a three-dimensional set of spatial data;
- registering at least a portion of the preparation site which exhibits a visible real surface feature by means of a camera located on the hand instrument at a specific distance from a machining tool and a display providing a video image;
- superimposing a computed surface feature for the target position of said hand instrument such that altering the position and angle of said hand instrument can cause a change in the position of said superimposed surface feature relatively to the visible real surface feature, and can, in particular, cause these two features to coincide, a horizon line being used as said surface feature.

2. A combination of a hand instrument (3), display means (12), and evaluating unit (13) for the creation of, or for machining, cavities, particularly bone cavities, which hand instrument (3) has a machining tool (4) and a camera (6) disposed at a known distance from the tip (7) of said machining tool (4), which hand instrument (3) is linked with said display means (12), and at least a portion of the preparation site showing a real visible surface feature is detected by said camera (6) and displayed, as a video image, on said display means (12), **characterized in that** said display means (12) display data collected by said evaluating unit (13) in the form of a horizon line (H) computed by said evaluating unit (13), and by changing the position and angle of said hand instrument (3), the position of said superimposed horizon line (H) can be changed relatively to the real visible horizon line and, in particular, be made to coincide therewith.

3. The combination as defined in claim 2, **characterized in that** said camera (6) has a depth of focus of from 5 to 30 mm and records a panorama view.

4. The combination as defined in claim 2 or claim 3, **characterized in that** said camera (6) is integrated **in that** end (5) of said instrument (3) which is near said machining tool (4).

5. The combination as defined in any one of claims 2 to 4, **characterized in that** illuminating means are provided for the purpose of illuminating that part of the surface which is relevant for registering and displaying said horizon line.

## Revendications

1. Procédé pour positionner dans la bonne orientation une pièce à main, munie d'un outil de traitement, sur un emplacement de préparation, comportant les étapes suivantes qui sont toutes mises en oeuvre avant le traitement proprement dit :
- calcul des caractéristiques de surface, qui sont fonction de la position, à partir d'un jeu de données tridimensionnelles de la surface de l'emplacement de préparation pour obtenir une position souhaitée d'un implant à introduire dans la cavité, la zone dans laquelle doit être ménagée la cavité étant disponible sous forme de jeu de données de volume tridimensionnelles ;
- enregistrement d'au moins une zone partielle de l'emplacement de préparation, comportant une caractéristique de surface réellement visible, au moyen d'une caméra montée sur la pièce à main à une distance prédéfinie d'un outil de traitement, et affichage sous forme d'image vidéo ;
- affichage à l'écran d'une caractéristique de surface calculée pour la position de consigne de la pièce à main, sachant que par une variation de la position et de l'inclinaison de la pièce à main, il est possible de faire varier l'orientation de la caractéristique de surface affichée à l'écran par rapport à la caractéristique de surface réellement visible, en particulier de l'amener en concordance, la caractéristique de surface utilisée étant une ligne horizontale.

2. Combinaison formée par une pièce à main (3), un dispositif d'affichage (12) et une unité d'analyse (13), pour la réalisation ou le traitement de cavités, en particulier des cavités osseuses, la pièce à main (3) étant munie d'un outil de traitement (4) et d'une caméra (6), dont la distance par rapport à la pointe (7) de l'outil de traitement (4) est prédéfinie, et la pièce à main (3) étant reliée au dispositif d'affichage (12), et au moins une zone partielle de l'emplacement de préparation, munie de la caractéristique de surface réellement visible, étant enregistrée au moyen de la caméra (6) et étant représentée sous forme d'image vidéo sur le dispositif d'affichage (12), **caractérisée en ce que** le dispositif d'affichage (12) affiche des données, mises à disposition par l'unité d'analyse (13), sous la forme d'une ligne horizontale (H), calculée par l'unité d'analyse (13), et **en ce que** par la variation de la position et de l'inclinaison de la pièce à main (3), il est possible de faire varier l'orientation de la ligne horizontale (H) affichée à l'écran par rapport à la ligne horizontale réellement visible, en particulier de l'amener en concordance.

3. Combinaison selon la revendication 2, **caractérisée en ce que** la caméra (6) possède une profondeur de netteté de 5 à 30 mm et enregistre une vue périphérique.

4. Combinaison selon la revendication 2 ou 3, **caractérisée en ce que** la caméra (6) est intégrée dans l'extrémité (5) de la pièce à main (3), orientée vers l'outil de traitement (4).

5. Combinaison selon l'une quelconque des revendications 2 à 4, **caractérisée en ce qu'**il est prévu des moyens lumineux, qui éclairent la partie de la surface, ayant de l'importance pour l'enregistrement et la représentation de la ligne horizontale.
